# EUROPEAN PATENT APPLICATION

(11) **EP 2 062 967 A1**
(43) Date of publication of application: **27.05.2009**
(21) Application number: 07022525.5
(22) Date of filing: 21.11.2007
(51) Int. Cl.: C12N 1/14, C12N 9/88, C12P 21/02, C12R 1/66

(54) **Genetically engineered aspergillus**

(71) Applicant: Technical University of Denmark, 2800 Lyngby (DK)
(72) Inventor: Nielsen, Jens, 2920 Charlottenlund (DK); Olsson, Lisbeth, 2800 Kgs. Lyngby (DK); Panagiotou, Gianni, 1657 Copenhagen V (DK); Regueira, Torsten Bak, 2300 Copenhagen S (DK)
(74) Representative: Benthin, Stig

(57) **Abstract**

Genetically modified *Aspergillus* strains having improved growth on xylose.

## Description

### FIELD OF THE INVENTION

The present invention relates to the field of genetic engineering and to the field of industrial biotechnology. Particularly the present invention relates to genetically engineered *Aspergillus* having improved ability to grow on xylose as carbon source.

### BACKGROUND OF THE INVENTION

Starch, glucose and molasses have traditionally been the main carbon sources (raw materials) in industrial fermentations. However, any carbon source could potentially be used as feedstock in a biotechnological process with aspergilli. Today increased focus is on harnessing the value of agricultural residues such as corn stover and wheat straw; materials that are rich in xylose (25 -40 % of the dry matter content). A prerequisite for making the introduction of new biomass streams economically attractive is to ensure efficient utilization of all sugars in the new biomass stream.

Filamentous fungi and fungal products are of remarkable importance to humans. The filamentous fungal genus *Aspergillus* is particularly important because it encompasses diverse species of great relevance within the medical and agricultural industry. The biotechnological importance of aspergilli may be illustrated by the use of *Aspergillus niger* for the industrial production of citric acid and recombinant proteins such as glycoamylases and lipases. Furthermore, *Aspergillus terreus* is used for the production of the secondary metabolite lovostatin, a cholesterol lowering-agent. An important fact is that the production of the above mentioned compounds is coupled to the growth of the aspergilli, i.e. enhanced growth results in a higher productivity of the compound of interest and a concomitant improvement of the production process. It is therefore important for *Aspergillus* strains to exhibit a high growth rate on the particular carbon source used in the industrial process.

From a commercial point of view, the fundamental knowledge on fungal genetics, biochemistry, and physiology of A. *nidulans* is invaluable for improving industrial processes. New and improved strains can be obtained by altering the properties of orphan filamentous fungal genes. Xylose is an important carbon source and the most abundant pentose sugar in hemicellulose of hardwoods and crop residues (ca. 25% of dry weight). Xylose is a major monosaccharide in lignocellulosic material, which is forecasted to a very important future carbon source for industrial processes in the area of industrial biotechnology.

Phosphoketolase (E.C. 4.1.2.9) catalyzes the reaction where D-xylulose 5-phosphate and inorganic phosphate is converted to acetyl phosphate and D-glyceraldehyde 3-phosphate. Phosphoketolase is known from a number of organisms, e.g. *Bifidobacterium* and *Lactobacillus.* In the fungal organisms *Penicillium* and *Aspergillus* there have been some indications and speculations of a phosphoketolase being present, see e.g. Process Biochemistry 2007, 42, 1050-1055).

Integration of genes from other organisms often results in unstable stains. Likewise, the integration of whole pathways tends to result in unstable strains and to increased complexity on determining the proper expression levels of the individual enzymes comprising this pathway.

WO 03/062430 discloses host cells transformed with a nucleic acid sequence encoding a eukaryotic xylose isomerase conferring the host with the ability to convert xylose to xylulose.

Appl. Environ. Microbiol. 2004, 70, 2892-2897 discloses metabolic engineering of the phosphoketolase pathway in *Saccharomyces cerevisiae.* By heterologous expression of phosphotransacetylase and acetaldehyde dehydrogenase in combination with the native phosphoketolase, a functional pentose phosphate pathway was installed in *Saccharomyces cerevisiae.*

WO 2006/009434 discloses host cells with the ability to directly isomerize xylose into xylulose for increasing the flux of the pentose phosphate pathway.

Metabolomics (2007, DOI: 10.1007/s11306-007-0061-7) discloses the induction of the phosphoketolase pathway in *Aspergillus nidulans* cultivations.

### SUMMARY OF THE INVENTION

It has surprisingly been found that aspergilli with higher expression levels of phosphoketolase exhibit increased growth rate on carbon sources such as xylose.
The present invention provides the aspergilli based fermentation industry with improved microorganisms that are capable of fast and efficient utilisation of an additional carbon source (xylose). The invention is directed to the improvement of growth and can therefore be used for the production of a wide range of products originating from aspergilli.
Integration of genes from other organisms often results in unstable stains which are therefore unsuitable for use in the fermentation industry. Also it is usually necessary to introduce several different genes in order to significantly redirect the carbon flow to the desired metabolic pathways. According to the present invention heterologous genes are not necessary as aspergilli contain a native phosphoketolase gene which can be used for the synthesis of the phosphoketolase protein.

In addition, the phosphoketolase pathway can be induced in a number of different aspergilli that are currently used in the industry. Thus, the invention can be used to improve many different organisms and processes. The beneficial effects of phosphoketolase is not only limited to the growth of A. *nidulans* on xylose but also to growth on diverse carbon sources such as glycerol and ethanol. Phosophoketolase is bringing the future vision of a bio-refinery - an integrated process platform that converts biomass derived feed streams to a diversified portfolio of product streams, adjusted according to market demands - one step closer to reality.

### DETAILED DESCRIPTION OF THE INVENTION

The main advantage of the proposed strategy to increase the specific growth rate is that it is based on the overexpression of a single gene (phosphoketolase) in an Aspergillus.

The invention also has the potential to improve growth when cells are grown on other carbon sources than xylose, e.g. other pentoses, glycerol and ethanol.

In a first aspect the present invention provides an isolated *Aspergillus* which is capable of utilizing xylose characterized in said *Aspergillus* overexpressing an enzyme which has phosphoketolase activity.
We have found that Aspergillus comprise a native gene encoding phosphoketolase, which gene may be utilized for overexpression of the phosphoketolase protein and subsequently the phosphoketolase metabolic pathway. The term "overexpression" as used herein is intended to mean expression of a polypeptide beyond the level which is normally expressed by said microorganism. The overexpression could be depicted in the difference at the mRNA level, at the protein level, at the activity level of the enzyme or at the flux through the phosphoketolase pathway between the constructed strain and the wild type Aspergillus. An Aspergillus may be genetically modified, e.g. turned into a mutant, to overexpress phosphoketolase in a number of ways. Firstly, the Aspergillus of interest may be subjected to random mutagenesis, e.g. by irradiation with UV light or by chemical treatment with base analogs or alkylation agents, followed by selection in a medium comprising xylose as carbon source. Secondly, the *Aspergillus* of interest may be subject to recombinant DNA techniques to introduce one or more phosphoketolase genes under the control of a strong promoter in either the chromosome or in an extrachromosomal vector.
In one embodiment the isolated *Aspergillus* according to the invention overexpresses phosphoketolase due to the presence of at least one phosphoketolase gene integrated into the genome. The phosphoketolase gene may be a gene native to the *Aspergillus* or it may be a heterologous phosphoketolase gene.
In one embodiment the phosphoketolase has the following sequence :

MPGEVIERPNPAPKPSHVPDLVEKLIIPAQKTKLEKSDCDALHKYRRAAAYIAAGHWGTCPGLIL
VYSHLNYLIKKQNLD
MLYVVGPGHGAPGLLASLWLEGSLGKFYPQYTKDKEGLHNLISTFSTSAGLPSHINAETPGAIHE
GGELGYALSVSFGAV
MDNPDLIVTCVVGDGEAETGPTATSWHAIKYIDPAESGAVLPILHVNGFKISERTIFGCMDNREI
VCLFTGYGYQVRIVE
DLEDIDNDLHSAMSWAVEEIRNIQKAARSGKPIMKPQWPMIVLRTPKGWSGPKELHGQFIEGSFH
SHQVPLPNAKKDDEE
LQALQKWLSSYKPDELFTESGDVIDEILSIIPSDDKKLGMRPEAYKTHLPPDLPDWRQFCVKKGD
QFSAMKAIGSFIDQV
FVKNPHTVRLFSPDELESNKLSAALSHTGRNFQWDEFSNAKGGRVIEVLSEHLCQGFMQGYTLTG
RTGIFPSYESFLGII
HTMMVQYAKFAKMAKETAWHHDVSSINYIETSTWARQEHNGFSHQNPSFIGAVLKLKPYAARVYL

PPDANTFLTTLHHCL

KSKNYINLMVGSKQPTPVYLSPEEAESHCRAGASIFKFCSTDGGLRPDVVLVGIGVEVMFEVIKA

AAILRERCPELRVRV

VNVTDLFILENEGAHPHALKHEAFDNLFTEDRSIHFNYHGYVNELQGLLFGRPRLDRATIKGYKE

EGSTTTPFDMMLVNE

VSRYHVAKAAVTGGARFNEKVKLRHQELCSEFDHNIAETRKYIMNNHQDPEDTYNMPSFN

This phosphoketolase is encoded by the following coding sequence :

ATGCCAGGAGAAGTCATCGAGCGACCAAACCCGGCGCCAAAGCCGTCGCATGTCCCCGATTTAGT
AGAAAAACTCATAAT
TCCCGCGCAGAAGACGAAGCTGGAGAAAAGCGATTGTGATGCGTTGCACAAGTATCGTCGCGCGG
CGGCGTATATTGCTG
CTGGACACTGGGGAACATGTCCTGGGTTGATCCTCGTTTACTCTCACCTGAACTACCTGATCAAG
AAGCAGAACCTCGAT
ATGCTGTATGTTGTTGGGCCAGGGCATGGAGCACCGGGCTTGCTAGCATCGTTGTGGCTTGAGGG
CTCGCTGGGGAAGTT
TTATCCGCAGTATACCAAGGACAAGGAGGGTCTGCACAACCTCATCTCGACGTTCAGTACCAGTG
CCGGACTGCCTAGCC
ACATCAATGCTGAAACACCCGGCGCTATCCACGAAGGAGGAGAGCTGGGCTATGCGCTGTCTGTC
TCCTTCGGCGCAGTC
ATGGATAACCCCGATTTGATTGTAACATGTGTAGTTGGAGACGGAGAGGCGGAAACTGGTCCAAC
AGCCACATCATGGCA
TGCGATCAAGTACATCGATCCAGCTGAGTCAGGAGCCGTGCTTCCAATCCTGCATGTGAATGGCT
TCAAGATCAGTGAGC
GCACTATTTTTGGGTGCATGGATAACAGGGAGATAGTCTGCCTGTTCACAGGGTACGGGTATCAG
GTGCGCATTGTCGAG
GACCTCGAGGACATCGACAACGACCTTCACAGCGCTATGTCCTGGGCGGTTGAGGAAATCCGTAA
TATTCAGAAAGCAGC
GCGCTCCGGAAAGCCTATCATGAAGCCTCAATGGCCCATGATTGTCTTGCGAACGCCCAAGGGTT
GGTCAGGGCCGAAAG
AGCTGCATGGCCAGTTCATCGAAGGATCGTTCCACTCCCACCAGGTTCCCCTCCCTAATGCTAAG
AAAGACGATGAGGAG
CTCCAGGCTCTGCAGAAATGGCTTTCCTCTTACAAACCCGATGAGCTGTTTACCGAGTCTGGCGA
CGTTATCGACGAAAT
CCTATCCATTATTCCTTCGGATGATAAGAAACTCGGCATGAGACCCGAGGCCTACAAGACTCATC

TACCGCCGGACCTCC
CTGACTGGAGACAGTTCTGCGTGAAAAAGGGGATCAGTTCAGCGCAATGAAGGCCATTGGTAGC
TTCATCGACCAGGTT
TTCGTCAAGAACCCGCATACCGTCCGGTTATTCTCACCCGACGAGCTGGAAAGCAACAAGTTGAG
CGCTGCCCTATCACA
TACGGGAAGGAATTTCCAGTGGGATGAGTTCTCGAATGCAAAAGGTGGGCGGGTGATCGAGGTCC
TGAGTGAGCATTTGT
GTCAGGGCTTCATGCAGGGGTATACATTGACCGGCCGGACGGGCATCTTCCCATCATATGAGAGT
TTCTTGGGTATTATA
CATACCATGATGGTCCAGTATGCCAAGTTCGCAAAGATGGCTAAAGAAACGGCATGGCACCATGA
CGTGAGTAGTATCAA
CTACATCGAGACCAGCACCTGGGCCCGACAGGAGCACAATGGCTTCTCTCACCAAAATCCATCCT
TCATCGGCGCGGTTC
TCAAACTGAAGCCGTACGCCGCCCGCGTCTACCTGCCTCCCGACGCCAACACATTTCTTACCACT
TTGCACCACTGCCTG
AAATCAAAGAATTATATCAACCTCATGGTCGGCTCAAAGCAACCCACCCCAGTCTACCTGAGCCC
CGAGGAAGCGGAAAG
CCACTGCCGAGCCGGAGCCTCGATCTTCAAGTTCTGCAGTACCGACGGTGGGCTCCGCCCGGATG
TCGTACTCGTTGGAA
TCGGTGTTGAGGTCATGTTCGAAGTTATCAAGGCGGCAGCCATACTGCGAGAACGATGCCCTGAG
CTGCGTGTTCGTGTA
GTCAACGTGACGGATTTATTCATTCTCGAGAACGAGGGTGCCCACCCCCACGCCTTGAAGCACGA
GGCCTTCGACAACCT
CTTCACCGAGGATCGCTCCATCCATTTCAACTATCATGGATATGTGAACGAACTCCAGGGCCTGC
TCTTTGGCCGCCCTA
GGCTCGACCGGGCAACCATCAAGGGATATAAGGAAGAGGGAAGCACCACAACTCCATTTGACATG
ATGCTTGTGAATGAA
GTATCGCGGTACCACGTCGCGAAGGCAGCCGTCACGGGAGGAGCGAGGTTCAATGAGAAAGTCAA
GCTGCGGCACCAGGA
GCTTTGCTCTGAATTCGATCATAACATTGCTGAGACGCGCAAGTACATCATGAACAATCATCAAG
ATCCCGAAGACACAT ACAATATGCCCTCATTTAACTAG

In another embodiment the overexpression of the phosphoketolase activity is caused by an improved regulatory sequence for at least one phosphoketolase gene.
In yet another embodiment the isolated *Aspergillus* according to the present invention has a phosphoketolase activity, under cultivation conditions as set out in Example 1, which is at least twice the phosphoketolase activity of the corresponding *Aspergillus* that has not been modified to overexpress phosphoketolase. The phosphoketolase activity can be detected using a number of different enzymatic assays (Tanaka et al., 2002, Appl Microbiol Biotechnol, 60:160-167; Evans et al., 1984, Arch Microbiol, 139:48-52,; Marwoto et al., 2004, Appl Microbiol Biotechnol, 64:112-119)
In another embodiment the isolated *Aspergillus* has a specific growth rate on xylose of at least 0.16 h⁻¹, at least 0.18 h⁻¹, at least 0.19 h⁻¹, or at least 0.22 h⁻¹ when determined under the growth conditions set out in Example 1.

As phosphoketolase genes are available from a number of microorganisms, including various *Aspergillus* species, the isolated *Aspergillus* according to the present invention in a further embodiment is *A. niger, A. oryzae, A. terreus, A. nidulans, A. clavatus, A. flavus or A. fumigatus.*

The modified *Aspergillus* overexpressing phosphoketolase are particularly interesting when they also express a heterologous polypeptide. In this way readily available raw materials in the form of agricultural residues, such as corn stover and wheat straw, may be used as inexpensive carbon source for the industrial production of said polypeptides by fermentation.
In one embodiment of the invention the heterologous polypeptide is an enzyme or a therapeutic polypeptide.

### EXPRESSION OF HETEROLOGOUS POLYPEPTIDE

In another aspect the present invention provides a method of producing polypeptides in an Aspergillus host cell according to the invention, including heterologous polypeptides, which method comprises introducing into said host cell a nucleic acid sequence encoding the polypeptide of interest, modifying the host cell to overexpress phosphoketolase, cultivating the resultant host cell in a suitable growth medium, and recovering said polypeptide of interest. Introduction of the nucleic acid sequence encoding the polypeptide of interest into the Aspergillus may be done before modifying the Aspergillus to overexpress phosphoketolase, or it may be done after the said Aspergillus has been modified to overexpress phosphoketolase.

Thus, the host cell according to the invention must contain structural and regulatory genetic regions necessary for the expression of the polypeptide of interest. The nature of such structural and regulatory regions depends to a large extent on the product and the particular Aspergillus host strain. The genetic design of the host cell according to the invention may be accomplished by the person skilled in the art using standard recombinant DNA technology for the transformation or transfection of a host cell (see, e.g., Sambrook et al.).
The host cell may be modified by methods known in the art for the introduction of an appropriate cloning vehicle, i.e., a plasmid or a vector, comprising a DNA fragment encoding the desired polypeptide of interest. The DNA fragment of interest may be introduced into the host cell either as an autonomously replicating plasmid or integrated into the chromosome. The cloning vehicle may comprise one or more structural regions operable linked to one or more appropriate regulatory regions.
The structural regions are regions of nucleotide sequences encoding the polypeptide of interest. The regulatory regions include promoter regions comprising transcription and translation control sequences, terminator regions comprising stop signals, and polyadenylation regions. The promoter, i.e., a nucleotide sequence exhibiting a transcriptional activity in the host cell of choice, may be one derived from a gene encoding an extracellular or an intracellular polypeptide, such as an amylase, a glucoamylase, a protease, a lipase, a cellulase, a xylanase, an oxidoreductase, a pectinase, a cutinase, or a glycolytic enzyme. Examples of suitable promoters for directing the transcription of the nucleic acid constructs in the methods of the present invention are promoters obtained from the genes encoding Aspergillus oryzae TAKA amylase, Rhizomucor miehei aspartic proteinase, Aspergillus niger neutral alpha-amylase, Aspergillus niger acid stable alpha-amylase, Aspergillus niger or Aspergillus awamoriglucoamylase (glaA), Rhizomucor miehei lipase, Aspergillus oryzae alkaline protease, Aspergillus oryzae triose phosphate isomerase, Aspergillus nidulans acetamidase, Aspergillus oryzae acetamidase (amdS), Fusarium oxysporum trypsin-like protease (U.S. Pat. No. 4,288,627), and mutant, truncated, and hybrid promoters thereof. Another example of suitable promoters is the NA2-tpi promoters (a hybrid of the promoters from the genes encoding Aspergillus niger neutral alpha-amylase and Aspergillus oryzae triose phosphate isomerase), glucoamylase, and TAKA amylase promoters.
The cloning vehicle may also include a selectable marker. A selectable marker is a gene the product of which provides for biocide or viral resistance, resistance to heavy metals, prototrophy to auxotrophs, and the like. A selectable marker for use in a filamentous fungal host cell may be selected from the group including, but not limited to, amdS (acetamidase), argB (omithine carbamoyltransferase), bar (phosphinothricin acetyltransferase), hygB (hygromycin phosphotransferase), niaD (nitrate reductase), pyrG (orotidine-5'-phosphate decarboxylase), sC (sulfate adenyltransferase), and trpC (anthranilate synthase), as well as equivalents from other species. Particularly useful in an Aspergillus cell are the amdS and pyrG genes of Aspergillus nidulans or Aspergillus oryzae and the bar gene of Streptomyces hygroscopicus.
Furthermore, selection may be accomplished by co-transformation, wherein the transformation is carried out with a mixture of two vectors and the selection is made for one vector only.
The procedures used to ligate the DNA construct of the invention, the promoter, terminator and other elements, respectively, and to insert them into suitable cloning vehicles containing the information necessary for replication, are well known to persons skilled in the art (see, e.g., Sambrook et al., 1989). The mutant filamentous fungal cell is cultivated in a nutrient medium suitable for production of the polypeptide of interest using methods known in the art. For example, the cell may be cultivated by shake flask cultivation, small-scale or large-scale fermentation (including continuous, batch, fed-batch, or solid state fermentations) in laboratory or industrial fermentors using a suitable medium and under conditions allowing the heterologous polypeptide to be expressed and/or isolated. The cultivation takes place in a suitable nutrient medium comprising carbon (e.g. xylose) and nitrogen sources and inorganic salts, using procedures known in the art. Suitable media are available from commercial suppliers or may be prepared according to published compositions (e.g., in catalogues of the American Type Culture Collection). The secreted polypeptide can be recovered directly from the medium.
The polypeptide may be detected using methods known in the art that are specific for the polypeptide. These detection methods may include use of specific antibodies, formation of an enzyme product, disappearance of an enzyme substrate, or SDS-PAGE. For example, an enzyme assay may be used to determine the activity of the polypeptide. Procedures for determining enzyme activity are known in the art.
The resulting polypeptide can be isolated by methods known in the art. For example, the polypeptide may be isolated from the nutrient medium by conventional procedures including, but not limited to, centrifugation, filtration, extraction, spray drying, evaporation, or precipitation. The isolated polypeptide may then be further purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), or extraction (see, e.g., Protein Purification, J.-C. Janson and Lars Ryden, editors, VCH Publishers, New York, 1989).

The desired end product, i.e., the polypeptide of interest expressed by the Aspergillus host cell, may be any homologous or heterologous polypeptide. The polypeptide may be any polypeptide heterologous to the host cell. The term "polypeptide" is not meant herein to refer to a specific length of the encoded product and, therefore, encompasses protein, peptides and oligopeptides. The heterologous polypeptide may also be an engineered variant of a polypeptide. The term "heterologous polypeptide" is defined herein as a polypeptide, which is not native to the *Aspergillus* wherein it is expressed. The mutant filamentous fungal cell may contain one or more copies of the nucleic acid sequence encoding the heterologous polypeptide.
In the methods of the present invention, the host cell may also be used for the recombinant production of polypeptides, which are native to the cell. The native polypeptides may be recombinantly produced by, e.g., placing a gene encoding the polypeptide under the control of a different promoter to enhance expression of the polypeptide, to expedite export of a native polypeptide of interest outside the cell by use of a signal sequence, and to increase the copy number of a gene encoding the polypeptide normally produced by the cell. The present invention also encompasses, within the scope of the term "heterologous polypeptide", such recombinant production of homologous polypeptides, to the extent that such expression involves the use of genetic elements not native to the cell, or use of native elements which have been manipulated to function in a manner that do not normally occur in the host cell.
In a more specific embodiment, the product is a therapeutically active polypeptide, such as a hormone, e.g. insulin, growth hormone, glucagon, GLP-1 or somatostatin; an interleukin, e.g. interferon; a haematopoietic growth factor, e.g. PDGF (platelet derived growth factor), EPO (erythropoietin), or TPO (thrombopoietin); a protease, e.g. Factor VII, Factor VIII, urokinase, chymosin, or TPA; or serum albumin.
In another embodiment, the product is an enzyme of fungal or bacterial origin. The enzyme can be a glycosidase enzyme, e.g., an amylase, such as an alpha-amylase, a beta-amylase or a glucoamylase; a glucan alpha-1,4-glucosidase; an aminopeptidase; a carbohydrase; a carboxypeptidase; a catalase; a cellulase, such as an beta-1,4-endoglucanase or a beta-1,3(4)-endoglucanase; a cellulose-beta-1,4-cellobiosidase; a chitinase; a cutinase; a cyclodextrin glycosyltransferase; a deoxyribonuclease; a galactanase; a galactosidase, such as an alpha-galactosidase or a beta-galactosidase; an endoglucanase, such as an beta-1,3-endoglucanase, an alpha-1,3-endoglucanase, an beta-1,2-endoglucanase, or a beta-1,6-endoglucanase; a glucosidase, such as an alpha-glucosidase or a beta-glucosidase; an invertase; a laccase; a lipolytic enzyme, such as a lipase, an esterase, a phospholipase, or a lyso-phospholipase; a lyase or a pectate lyase; a mannase; a mannosidase; a polygalacturonase; a mutanase; an oxidase or an oxidoreductase, such as a peroxidase or a polyphenoloxidase; an oxygenase; a pectinase, an endo-peptidase or an exo-peptidase; a phytase; a polygalacturonase; a protease; a ribonuclease; a transglutaminase; and a xylanase, such as a beta-1,4-endoxylanase or a xylan-beta-1,3-endoxylosidase.
In another embodiment the product is a hybrid polypeptide, such as prochymosin, pro-trypsin-like proteases or a fusion between human serum albumin and a therapeutically active polypeptide. The heterologous polypeptide expressed by the host cell may, under suitable conditions, e.g., absence of substantial protease activities, also be a precursor protein such as a zymogen, a hybrid protein, a protein obtained as a pro sequence or a pre-pro sequence, or any other immature form.

In another aspect the present invention provides the use of an isolated *Aspergillus* for the production of a recombinant polypeptide.
In another aspect the present invention provides a method for production of a polypeptide using *Aspergillus* as host cell, said method comprising the steps :
a) cultivating an isolated Aspergillus according to the present invention in a culture medium comprising xylose, and
b) isolating said polypeptide from the spent culture medium.
   In one embodiment the contents of xylose in said medium is at least 20% of the carbohydrates in said medium, such as at least 25%, at least 30% or at least 40% of the carbohydrates in said medium.

The present invention is further illustrated with reference to the following examples, which should not in any way be construed as limiting the scope of the invention as defined in the description and in the claims.

### EXAMPLES

### Example 1 - preparation of Aspergillus nidulans overexpressing phosphoketolase.

### Part 1: GENETIC MODIFICATIONS

### Strains, Media and Plasmids

*Aspergillus nidulans* AR1 [*pyrG89, argB2; veA1*], IBT 27263, was used in all transformation and expression experiments. In order to overexpress phosphoketolase (*phk*) in *A. nidulans* AR1, the gene was cloned into an integrative vector based on the purchased plasmid, pBARGPE1 (M.L. Pall, J.P. Brunelli http://www.fgsc.net/fgn/pall.html), obtained from the FGSC, USA. Transformants were selected on minimal medium (MM) with the necessary supplements. MM: 10 g/L glucose; 10 mM NaNO₃; 0.52 g/L KCL; 0.52 g/L MgSO₄; 1.52 g/L KH₂PO₄; 4·10⁻⁴ g/L CuSO₄.5H₂O; 4·10⁻⁵ g/L Na₂B₂O₇·10H₂O; 8·10⁻⁴ g/L FeSO₄·7H₂O; 8·10⁻⁴ g/L MnSO₄·2H₂O; 8·10⁻⁴ g/L Na₂MoO₄·2H₂O; 8·10⁻⁴ g/L ZnSO₄·7H₂O.

### Construction of plasmids

Standard molecular biotechnology techniques were applied according to (Sambrook et al, 2001), and final constructs were verified by sequencing. *Transformation ofA. nidulans AR1*
Genetic transformation of A. *nidulans* AR1 protoplasts was basically performed as previously described (Johnstone et al., 1985), except that protoplasting was achieved by using the enzyme, Glucanex, (Novozymes A/S), at a concentration of 40 mg/ml in protoplasting buffer. *Pvul* was used for linearization of the plasmid as only one restriction site is present in the vector and which is located in the ampicillin resistance gene.
Transformants were purified by streaking out spores to obtain single colonies on selective minimal medium and incubated at 37°C for 3-4 days. The resulting recombinants were further purified twice by streaking out spores on fresh plates with selective medium.

### Results:

The gene was cloned into a modified form of the vector pBARGPE1. In this modified vector the genes were expressed under control of the strong constitutive glycolysis-promoter from *glyceraldehyde-3-phosphate dehydrogenase* (*gpdA*) of A. *nidulans.*

### Construction of Plasmids

Several plasmids were constructed and all were based on the pBARGPE1 vector, which contains the *gpdA* promoter from *A. nidulans,* linked to a multiple cloning site (MCS) and the TrpC terminator. The plasmid also carries the BASTA resistance gene, bar, which however was not used as a selective marker in this study, due to the availability of more stringent auxotrophic markers in A. nidulans. *PyrG* from *Aspergillus funigatus* was used as selection marker. All constructed plasmids were verified by sequencing.
Construction of pTr02PHK and Heterologous Expression of *phk* in *A*. *nidulans*
The putative PHK gene from *A. nidulans* (XP_662517) was amplified from genomic DNA with PCR using the Fphk_FW/RV primers.
Transformants were selected on uracil and uridine deficient MM medium and were indistuingshable from the original *A. nidulans* AR1 strain with respect to morphology when growing on supplemented MM agar plates.
The expression analysis (Northern blot) clearly indicated a strong overexpression of the putative phosphoketolase gene in the transformant compared to the reference strain (data not shown). To further verify that the overexpressed gene is actually coding for phosphoketolase the wild type (A4) and the mutant (AR1*phk*GP74) were cultivated on glucose in the presence of 1 mM of iodo acetate. While iodo acetate prohibited growth of the A4 cells (specific growth rate of 0.01 h⁻¹) had almost no effect on the growth rate of the AR1*phk*GP74 mutant (specific growth rate of 0.11 h⁻¹). However, the growth of the AR1*phk*GP74 mutant was paused after half of the carbon source was consumed indicating a deficiency of the cells.

### Part 2: PHYSIOLOGICAL CHARACTERIZATION

### Cultivation conditions

For all the A. *nidulans* cultivations a defined medium containing trace metal elements was used. The medium used had the following composition: 15 g (NH₄)₂SO₄ I⁻¹, 3 g KH₂PO₄ I⁻¹, 2 g MgSO₄.7H₂O I⁻¹, 2 g NaCl I⁻¹, 0.2 g CaCl₂ I⁻¹ and 1 ml trace element solution I⁻¹. Trace element solution composition (per litre): 14.3 g ZnSO₄.7H₂O, 13.8 g FeSO₄.7H₂O and 2.5 g CuSO₄.5H₂O. Arginine, 0.7 g/L, was added to the auxotrophic strains by sterile filtration. The carbon sources used was xylose (20 g I⁻¹). To determine the physiological characteristics cultivations were performed in well-controlled 1.5 I bioreactors with a working volume of 1.2 I. The bioreactors were equipped with two disc-turbine impellers rotating at 350 r.p.m. The pH was kept constant at 5.5 by addition of 2 M NaOH or HCl and the temperature was maintained at 30 °C. Air was used for sparging the bioreactor at a constant flow rate of 1.0 vvm (volume of gas per volume of liquid per minute).

A set of experiments was carried out to investigate the metabolism of the *A. nidulans* wild type (A4) and the *A. nidulans* AR1*phk*GP74 strain (mutant with overxpressed phosphoketolase). Substrate and end-products were determined during the growth of the wild type and the mutant on xylose. The maximum specific growth rate and the biomass yield were determined from the growth phase, which was fully aerobic. During this step biomass was the main product formed. Significant effect on the growth rate, caused by the overexpression of the phosphoketolase gene, was observed when the cells were cultivated on xylose. The AR1*phk*GP74 strain could grow 1.3 times faster than the wild type reaching a specific growth rate of 0.19 h⁻¹ compared to the 0.16 h⁻¹ of the A4 strain.The biomass yield was similar for the two strains and in the range of the values that were obtained also with glucose as carbon source (0.46 g/g for the A4 and 0.47 for the AR1*phk*GP74).

### Example 2. Preparation of a mutant Aspergillus nidulans overexpressing phosphoketolase.

Classical strain improvement relying on UV radiation or chemical mutagenesis (base analog mutagens, alkylators, nitrous acid) can be used to effect overexpression of phosphoketolase. In such an approach *Aspergillus nidulans* mutants with a higher specific growth rate on xylose are selected by serial transfer in shake flasks. For serial transfer experiments *Aspergillus nidulans* is cultivated in the presence of xylose as carbon source in a minimal or complex medium. When the growth reaches the exponential phase the culture is used to inoculate a new shake flask and the procedure will be repeated until cells with improved growth rate are obtained.

### Example 3. Identification of the phosphoketolase gene in other aspergilli

In order to reveal if the phosphoketolase from *Aspergillus nidulans* has homologs throughout the Aspergilli genus a BLASTP search was performed. Using the BLAST utility of the BROAD Institute a comparison between the *Aspergillus nidulans* XP_662517 protein query *(Aspergillus nidulans*) and the protein databases of *Aspergillus niger, oryzae, fumigatus, flavus, fischeri, terreus,* and *clavatus* was applied. In Table 1 it is seen that significant hits were obtained for all the Aspergilli sp., and these hits were always phosphoketolases, in the cases where the protein was known. For *Aspergillus niger* and *Aspergillus oryzae* the protein that shows the highest similarity with the phosphoketolase from *Aspergillus nidulans* has not been identified as phosphoketolase before. However, the similiraty values (88% and 86% similarity for *Aspergillus niger* and *Aspergillus oryzae,* respectively, and 80% and 78% identity for *Aspergillus niger* and *Aspergillus oryzae,* respectively) give strong confidence that the above proteins are actually phosphoketolases.

**Table 1.**

| Blast Output | |
|---|---|
| gi\|67537486\|ref\|xp_662517.1\|hypothetical protein AN4913.2[Aspergillus nidulans FGSC A4] | |
| Summary of Hits by Genome | |
| A. fumigatus | 2 |
| A. flavus | 2 |
| N. fischeri | 2 |
| A. terreus | 2 |
| A. nidulans | 1 |
| A. oryzae | 2 |
| A. clavatus | 2 |
| A. niger | 1 |

| [Show All Alignments] [Hide All Alignments] [View Raw Output] | | | | | |
|---|---|---|---|---|---|
| Target | Score (Bits) | Expect | Alignment length | Identities | Positives |
| ► A. nidulans: AN4913.3: conserved hypothetical protein | 1581.23 | 0.0 | 768 | 768 | 768 |
| ► A. niger: est_GW1_C_21132: est_GW1_C_21132 | 1318.91 | 0.0 | 794 | 643 | 699 |
| ► A. oryzae: AO090003000625: Unknown | 1308.89 | 0.0 | 806 | 636 | 700 |
| ► N. fischeri: NFIA_066490: null | 1291.56 | 0.0 | 794 | 626 | 688 |
| ► A. davatus: ACLA_038690: null | 1287.71 | 0.0 | 794 | 622 | 689 |
| ► A. terreus: ATEG_04606.1: conserved hppothetical protein | 1275.38 | 0.0 | 794 | 620 | 685 |
| ► A. flavus: AFL2G_02377.2: conserved hypothetical protein | 1201.81 | 0.0 | 794 | 592 | 653 |
| ► A. fumigatus: Afu3g10760: D-xylulose 5-phosphate/D-fructose 6-phosphate phosphoketolase | 1172.53 | 0.0 | 686 | 561 | 619 |
| ► A.terreus:ATEG_07454.1: xylulose-5-phosphate phosphoketolase | 591.652 | 0.0 | 725 | 297 | 438 |
| ► A. oryzae: A0090012000711: Unknown | 585.104 | 0.0 | 717 | 292 | 436 |
| A. flavus: AFL2G_03586.2: xylulose-5-phosphate phosphoketolase | 585.104 | 0.0 | 717 | 292 | 436 |
| ► A. davatus: ACLA_064080: null | 577.4 | 0.0 | 719 | 291 | 432 |
| ► N.fischeri:NFLA_000900:null | 579.711 | 0.0 | 692 | 285 | 425 |
| ► A. fumigatus: Afu3g00370:D-xylulose 5-phosphate/D-fructose 6-phosphate phosphoketolase | 576.63 | 0.0 | 692 | 284 | 425 |

Further evidence that the phosphoketolase protein exists also in *Aspergillus niger* and *Aspergillus nidulans* was obtained by identifying the position of the genes coding for phosphoketolase in their genome. In the case of *Aspergillus nidulans* the gene coding for phosphoketolase is only 410 bp from the coding region for acetate kinase. Acetate kinase is converting the product of the the reaction that phosphoketolase is catalyzing, acetyl-P to acetate, and would be expected that their localization in the genome will be very close. Similarly, in *Aspergillus niger* this distance is 438 bp and in *Aspergillus oryzae* is 494 bp. Taking into account the average size of the genes in Aspergilli (1572 bp for *Aspergillus niger)* we can say that the genomic space is not enough for another gene to be in between.

## Claims

1. An isolated *Aspergillus* which is capable of utilizing xylose **characterized in** said *Aspergillus* overexpressing an enzyme which has phosphoketolase activity.

2. The isolated *Aspergillus* according to claim 1, which is a mutant.

3. The isolated *Aspergillus* according to any of the preceding claims, wherein overexpression of the phosphoketolase activity is caused by integration of at least one phosphoketolase gene.

4. The isolated *Aspergillus* according to any of the preceding claims, wherein said phosphoketolase gene is native to said *Aspergillus.*

5. The isolated *Aspergillus* according to any of the preceding claims, wherein overexpression of the phosphoketolase activity is caused by improving the regulatory sequence for at least one phosphoketolase gene.

6. The isolated *Aspergillus* according to any of the preceding claims, which under cultivation conditions as set out in Example 1 has a phosphoketolase activity which is at least twice the phosphoketolase activity of the corresponding Aspergillus that has not been modified to overexpress phosphoketolase.

7. The isolated *Aspergillus* according to any of the preceding claims, which has a specific growth rate on xylose of at least 0.16 h⁻¹, at least 0.18 h⁻¹, at least 0.19 h⁻¹, or at least 0.22 h⁻¹ when determined under the growth conditions set out in Example 1.

8. The isolated *Aspergillus* according to any of the preceding claims, which is *A. niger, A. oryzae, A. terreus, A. nidulans, A. clavatus, A. flavus or A. fumigatus.*

9. The isolated *Aspergillus* according to any of the preceding claims, which expresses a heterologous polypeptide.

10. The isolated *Aspergillus* according to claim 9, wherein said heterologous polypeptide is an enzyme or a therapeutic polypeptide.

11. Use of an isolated *Aspergillus* according to any of the preceding claims for the production of a recombinant polypeptide.

12. Method for production of a polypeptide using *Aspergillus* as host cell, said method comprising the steps :
a) cultivating an isolated Aspergillus according to any of claims 1-10 in a culture medium comprising xylose, and
b) isolating said polypeptide from the spent culture medium.

13. The method according to claim 12, wherein the contents of xylose in said medium is at least 20% of the carbohydrates in said medium.
